# EUROPEAN PATENT APPLICATION

(11) **EP 1 782 808 A1**
(43) Date of publication of application: **09.05.2007**
(21) Application number: 05766341.1
(22) Date of filing: 21.07.2005
(51) Int. Cl.: A61K 31/426, A61P 11/00, A61P 29/00, C07D 277/24

(54) **PREVENTIVE OR THERAPEUTIC AGENT FOR CHRONIC INFLAMMATORY LUNG DISEASE**

(30) Priority: 22.07.2004 JP 2004213803
(71) Applicant: KAKEN PHARMACEUTICAL CO., LTD., Bunkyo-ku, Tokyo 113-8650 (JP)
(72) Inventor: OKUDA, Toshiaki c/o KAKEN PHARMACEUTICAL CO., LTD., Kyoto-shi, Kyoto 6078042 (JP); KUROKAWA, Shigeo c/o KAKEN PHARMACEUTICAL CO., LTD, Kyoto-shi, Kyoto 6078042 (JP); MURATA, Takahiko c/o KAKEN PHARMACEUTICAL CO., LTD, Kyoto-shi, Kyoto 6078042 (JP); AMAKAWA, M. c/o KAKEN PHARMACEUTICAL CO., LTD., Kyoto-shi, Kyoto 6078042 (JP); NAKAMURA,Ts. c/o KAKEN PHARMACEUTICAL CO., LTD., Kyoto-shi, Kyoto 6078042 (JP)
(74) Representative: Keen, Celia Mary
(86) International application number: PCT/JP2005/013393
(87) International publication number: WO 2006/009209

(57) **Abstract**

Not only acute or chronic interstitial pneumonia and plumonary fibrosis but also chronic obstructive plumonary diseases, such as plumonary emphysema and chronic bronchitis, can be effectively prevented or treated by inhalation administration to the lung tissue of 2-{N-[4-(4-chlorobenzenesulfonylamino)butyl]-N-{3-[(4-isopropyl 2-thiazolyl)methoxy]benzyl}}-sulfamoylbenzoic acid or a pharmacologically acceptable salt thereof.

## Description

### TECHNICAL FIELD

The present invention relates to a medicament preventing or treating a chronic inflammatory pulmonary disease such as interstitial pneumonia, pulmonary fibrosis or chronic obstructive pulmonary diseases including pulmonary emphysema and chronic bronchitis, and containing 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof, as an active ingredient.

### BACKGROUND ART

Respiratory diseases are classified into interstitial pneumonia, chronic obstructive pulmonary disease (COPD), respiratory infection, allergic asthma, and pulmonary carcinoma, etc. Of them, interstitial pneumonia and COPD may be referred to as chronic inflammatory pulmonary diseases in which macrophages and neutrophils are mainly involved.
Interstitial pneumonia is a pulmonary disease mainly characterized by an inflammatory lesion of the pulmonary interstitial tissue and clinically divided into acute interstitial pneumonia and chronic interstitial pneumonia. Acute interstitial pneumonia (AIP) is a pulmonary disease characterized by acute respiratory failure and high lethality.
Chronic interstitial pneumonia (CIP) is a disease characterized by chronic, progressive cough and respiratory failure and accompanied by progressive pulmonary fibrosis (PF). When a causal substance of some kind mobilizes inflammatory cells and lymphocytes to the alveolar septum, an acute inflammation is induced. If the inflammation lasts long, the alveolar septum and the pulmonary alveoli are broken, with the result that the inflammation leads to chronic inflammation. In the following repairing process for the inflammation, conceivably excessive proliferation of the connective tissue develops into PF.
Interstitial pneumonia as mentioned above is classified into secondary interstitial pneumonia, which is induced by an apparent cause including an autoimmune disease such as collagen disease, a response due to infectious disease, side effects caused by an anticancer drug such as bleomycin (BLM) and radioactive rays, and inhalation of a foreign substance, and idiopathic interstitial pneumonia, which is, in most cases, induced by unknown causes.
In the cases of the secondary interstitial pneumonia induced by apparent causes, the disease can be ameliorated or further progression of the disease can be suppressed by just eliminating the cause. In contrast, there is no established therapy for idiopathic interstitial pneumonia. However, to temporalily ease the inflammation, steroids and an immune suppressive agents have been used. However, these agents presently have problems. In addition to side effects, a sufficient effect cannot be obtained depending on the progression of pathosis.

COPD is characterized by obstructive ventilatory impairment induced by chronic bronchitis, pulmonary emphysema or combination of them. It is a disease that slowly and irreversibly progresses and has a high incidence rate and lethality throughout the world. The chronic bronchitis is a disease with a state of increase in chronically or repeatedly expectorated respiratory secretions which continues at least for 2 years or more. The pulmonary emphysema is a disease accompanying abnormally enlarged peripheral air-spaces distal from the terminal bronchi and alveolar septal destruction. When tracheal epithelial cells and macrophages are activated by smoking and air pollutants, they produce/liberate cytokines and chemokines. Then, inflammatory cells such as macrophages, neutrophils and CD8 positive cells accumulate and activate in the lung. The inflammatory cells excessively produce various types of proteases, oxidants and lipid mediators. As a result, it is conceived that the connective tissue is broken and secretion of the mucus is accelerated.

At present, as a medication for COPD, drugs with a bronchodilation action, such as a β₂ stimulatory agent, anticholinergic agent, and theophylline preparation, are used for temporarily preventing or suppressing symptoms. Furthermore, in order to suppress inflammation, steroids are used. However, some documents have reported that according to investigations carried out in large-scale clinical tests, steroids failed to improve deterioration of the pulmonary function for the long term (Non-patent documents 1, 2).

As described above, although interstitial pneumonia and COPD are inflammatory pathosis, there are no medicaments capable of suppressing the inflammation. Steroids are not useful for these disease, because the pathosis is neutrophilic inflammation. Neutrophils and macrophages are activated by stimulation of various mediators such as foreign substances, prostanoids, cytokines and chemokines. When activated, they produce active oxygen and release not only proteases such as elastase but also cytokines to augment inflammatory response. In the circumstances, targeting various types of cytokines such as interleukin (IL)-1, interleukin (IL)-8, a tumor necrosis factor (TNF)-α, monocyte chemotactic parameter (MCP)-1, and leukotoriene (LT)B₄, and inflammatory mediators such as active enzymes, a preventive/therapeutic agent has been investigated.
The peripheral blood and bronchoalveolar lavage fluid of a pulmonary fibrosis patient contain large amounts of lipid mediators, i.e., arachidonic acid metabolites such as peptide leukotoriene (pLT), prostaglandin (PG) and thromboxane B₂ (TXB₂), suggesting that they participate in setting up the inflammation (Non-patent document 3). In BLM-induced pulmonary fibrosis mice, whose pathology of the lung is resembled to that of pulmonary fibrosis patients, the concentrations of LTB₄ and peptide leukotoriene (LTC₄, LTD₄ and LTE₄) are elevated in the bronchoalveolar lavage fluid. These mediators are produced by an enzyme, lipoxygenase. Lipoxygenase inhibitors are found to suppress development of pulmonary fibrosis. Therefore, it has been elucidated that these mediators acceleratively participate in onset of the disease (Non-patent document 4). Furthermore, there is a report that, in the BLM-induced pulmonary fibrosis model, mice whose the phospholipase A₂ gene initiating the arachidonic-acid cascade is destroyed demonstrated that pulmonary fibrosis is suppressed (Non-patent document 5). Moreover, there is a report that, in the BLM-induced pulmonary fibrosis model, a thromboxane synthase inhibitor suppresses the expression of collagen mRNA (Non-patent document 6). However, there are no reports as to whether or not a preventive/therapeutic effect on interstitial pneumonia can be produced by direct inhibition of LTB₄, pLT and arachidonic acid metabolites.
The bronchoalveolar lavage fluid in COPD patients contains large amounts of LTB₄ and IL-8 and the production of TNF-α is significantly increased by leukocytes in blood. An aqueous extract from cigarette smoke, which is a cause of COPD, accelerates production of chemotactic factors for neutrophils and macrophages from pulmonary fibroblasts. These mediators include neutrophils migration to an inflammatory site to exacerbate the inflammation. On the other hand, LTD₄ and a metabolite of TXA₂, namely, TXB₂, are not found in the bronchoalveolar lavage fluid. In addition, there is no report directly informing that they are involved in COPD. According to the reports, LTD₄-receptor antagonists such as montelukast and zafirlukast, or a TXA₂-receptor antagonist such as seratorodast, improves the respiratory function of COPD patients, thereby improving QOL (Non-patent documents 7, 8 and patent document 1). However, the effectiveness of these agents in preventing or treating the inflammatory pathosis of COPD have not yet been proven.

As a compound with antagonist activities against receptors for both mediators, LTD₄ and TXA₂, a 2-sulfamoylbenzoic acid derivative, such as 2-{N-[4-(4-chlorobenzenesulfonylamino)butyl]-N-{3-[(4-isopropyl-2-thiazolyl)methoxy]benzyl}}sulfamoylbenzoic acid has been known (patent document 2). However, it has not been elucidated that these compounds can be used as a preventive or therapeutic agent for a chronic inflammatory pulmonary disease such as interstitial pneumonia and COPD.
Patent document 1: WO 00/30633 pamphlet
Patent document 2: WO 98/57935 pamphlet
Non-patent document 1: Pauwels RA. et al., N Engl J Med, 340: 1948-1953 (1999)
Non-patent document 2: Vestbo J. et al., Lancet, 353: 1819-1823 (1999)
Non-patent document 3: S. Kitamura et al., Journal of Internal medicine 80: 1508-1513 (1991)
Non-patent document 4: Nagase T. et al., Nature Medicine, 8: 480-484 (2002)
Non-patent document 5: Golden M. et al., Am J Respir Crit Care Med, 165: 229-235 (2002)
Non-patent document 6: Sato Y. et al., Biomed Pharmacother, 58: 381-387 (2004)
Non-patent document 7: Rubinstein I. et al., Repir Med, 98:134-138 (2004)
Non-patent document 8: Luis J. et al., Pulm Pharmacol Ther, 16: 305-311 (2003)

### DISCLOSURE OF THE INVENTION

In view of the prior art as mentioned above, an object of the present invention is to provide a preventive or therapeutic agent for a chronic inflammatory pulmonary disease such as interstitial pneumonia and COPD.

The present inventors have intensively investigated with the view to obtaining a preventive or therapeutic agent for a chronic inflammatory pulmonary disease such as interstitial pneumonia and COPD. As a result, they found that 2-{N-[4-(4-chlorobenzenesulfonylamino)butyl]-N-{3-[(4-isopropyl-2-thiazolyl)methoxy]benzyl}}sulfamoylbenzoic acid, which is known to a dual antagonist against LTD₄/TXA₂, suppressed migration of neutrophils induced by LTB₄, which is an important promoting factor of chronic inflammatory pulmonary diseases. They further found that it significantly ameliorated medical conditions in an interstitial pneumonia model and COPD models. Based on these findings, the present invention was achieved.

Accordingly, the present invention relates to a medicament containing 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof, as an active ingredient, for preventing or treating a chronic inflammatory pulmonary disease.
Furthermore, the present invention relates to use of 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof, for producing a medicament for preventing or treating a chronic inflammatory pulmonary disease.
Moreover, the present invention relates to a method of preventing or treating a chronic inflammatory pulmonary disease in mammals including human, comprising administering, to the mammal, 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof.

As shown in Examples later described, an active ingredient of the medicament of the present invention, i.e., 2-{N-[4-(4-chlorobenzenesulfonylamino)butyl]-N-{3-[(4-isopropyl-2-thiazolyl)methoxy]benzyl}}sulfamoylbenzoic acid (hereinafter sometimes referred to as "KP-496"), showed a suppressive effect on LTB₄-induced human neutrophil migration in a concentration depending manner.

As shown in Examples later described, an active ingredient of the medicament of the present invention, i.e., KP-496, significantly suppressed infiltration of inflammatory cells such as neutrophils, eosinophils and lymphocytes induced by BLM in a murine BLM-induced pulmonary fibrosis model. Furthermore, with respect to histopathologic change of the lung tissue induced by BLM, the active ingredient of the present invention obviously reduced acute inflammation and chronic inflammation and clearly suppressed progression to the final pathologic stage of the inflammation model, that is, fibrosis. Moreover, it significantly decreased a marker specific to a component of fibrosis, i.e., hydroxyproline, in the lung tissue. These results revealed that the active ingredient of the present invention can reduce the BLM-induced acute inflammation of the early stage in the lung tissue and suppress the following chronic inflammation and fibrogenesis as well. Therefore, the active ingredient of the present invention is extremely effective as a preventive or therapeutic agent for acute and chronic interstitial pneumonia or pulmonary fibrosis.

As shown in Examples later described, an active ingredient of the medicament of the present invention, i.e., KP-496, exhibited an ameliorating effect on generation of pulmonary emphysema in a porcine pancreatic elastase (PPE)-induced emphysema model in mice. Furthermore, the active ingredient of the present invention showed an ameliorating effect on formation of pulmonary emphysema in an experiment performed by changing initiation time of administration. Moreover, the active ingredient of the present invention reduced excessive secretion of the mucus associated with the inflammation in the intrapulmonary bronchus (lobar bronchus) in a vanadium pentoxide-induced bronchitis model in mice.
From these results, the active ingredient of the present invention can ameliorate formation of PPE-induced pulmonary emphysema and suppress production of the mucus in the vanadium pentoxide-induced bronchitis, and is thus extremely useful as a preventive or therapeutic agent for COPD.

### BRIEF DESCRIPTION OF THE DRAWINGS

Fig. 1 shows the effects of KP-496, montelukast, pranlukast, and seratorodast on LTB₄-induced human neutrophil migration;
Fig. 2 shows the effect of KP-496 on the total number of cells in BALF in a murine model of BLM-induced pulmonary fibrosis;
Fig. 3 shows the effect of KP-496 on the number of alveolar macrophages in BALF in a murine model of BLM-induced pulmonary fibrosis;
Fig. 4 shows the effect of KP-496 on the number of neutrophils in BALF in a murine model of BLM-induced pulmonary fibrosis;
Fig. 5 shows the effect of KP-496 on the number of eosinophils in BALF in a murine model of BLM-induced pulmonary fibrosis;
Fig. 6 shows the effect of KP-496 on the number of lymphocytes in BALF in a murine model of BLM-induced pulmonary fibrosis;
Fig. 7 shows the effect of KP-496 on the hydroxyproline content of the lung in a murine model of BLM-induced pulmonary fibrosis;
Fig. 8 shows the effect of KP-496 on the lung damage score in a murine model of BLM-induced pulmonary fibrosis, where Fig. A shows the effect of KP-496 on acute inflammatory lesions on the 7th day after BLM administration; Fig. B the effect of KP-496 on chronic inflammatory lesions on the 21st day after BLM administration; and Fig. C the effect of KP-496 on fibrosis lesions on the 21st day after BLM administration;
Fig. 9 shows the effect of KP-496 on body weight change of mice in a PPE-induced pulmonary emphysema model;
Fig. 10 shows the effect of KP-496 on the total number of cells in BALF of mice in a PPE-induced pulmonary emphysema model;
Fig. 11 shows the effect of KP-496 on the number of macrophages in BALF of mice in a PPE-induced pulmonary emphysema model;
Fig. 12 shows the effect of KP-496 on the number of neutrophils in BALF of mice in a PPE-induced pulmonary emphysema model;
Fig. 13 shows the effect of KP-496 on the number of eosinophils in BALF of mice in a PPE-induced pulmonary emphysema model;
Fig. 14 shows the effect of KP-496 on the weight of the lung of mice in a PPE-induced pulmonary emphysema model;
Fig. 15 shows the effect of KP-496 on the lung damage score of mice in a PPE-induced pulmonary emphysema model;
Fig. 16 shows the effect of KP-496 on the average of alveolar-space area of mice in a PPE-induced pulmonary emphysema model;
Fig. 17 shows the effect of KP-496 on weights of the organs: the liver, spleen and thymus gland of mice in a PPE-induced pulmonary emphysema model, where Figs. A, B and C are graphs regarding the liver, spleen and thymus gland; and
Fig. 18 shows the effect of KP-496 on mucus production in the intrapulmonary bronchial epithelium of mice in a vanadium pentoxide-induced bronchitis model.

### BEST MODE FOR CARRYING OUT THE INVENTION

The active ingredient of a medicament for preventing or treating a chronic inflammatory pulmonary disease of the present invention is 2-{N-[4-(4-chlorobenzenesulfonylamino)butyl]-N-{3-[(4-isopropyl-2-thiazolyl)methoxy]benzyl}}sulfamoylbenzoic acid (KP-496) or a pharmaceutically acceptable salt thereof. Examples of such a salt include salts of a metal such as sodium, potassium, magnesium, and calcium; and organic salts such as trimethylamine, triethylamine, pyridine, picoline, N-methylpyrrolidine, N-methylpiperidine and N-methylmorpholine. These active ingredients may take the form of a hydrate. These active ingredients can be produced by chemical synthetic methods described in WO98/57935.
As a chronic inflammatory disease, interstitial pneumonia, targeted for the medicament of the present invention, more specifically, acute or chronic interstitial pneumonia may be mentioned. Furthermore, secondary interstitial pneumonia, which is induced by an apparent cause including an autoimmune disease such as collagen disease, infectious disease, radioactive rays, a drug such as bleomycin or inhalation of a foreign substance, and idiopathic interstitial pneumonia induced by an unknown cause may be mentioned.
As the chronic inflammatory pulmonary disease COPD, pulmonary emphysema or chronic bronchitis may be mentioned. COPD is caused by smoking or air pollutants. Examples of the air pollutants include smoking gas, exhaust gas, inorganic dust, and sooty smoke.

The active ingredient of the present invention can be administered singly as it is or in the form of various preparations formed by general preparation methods. Examples of the preparations that can be administered include oral preparations such as a tablet, capsule, granule, fine grain, powder, liquid and syrup; and parenteral preparations such as injection, nose drop, drip infusion, inhalant, transdermal absorption agent and adhesive skin patch. Particularly, the active ingredient of the present invention is preferably formed into an inhalant and administered by inhalation through the nose or oral cavity. The inhalant is preferably prepared by molding the active ingredient into the form of powder, fine grain, granular agent or the like in accordance with a conventional method and encapsulating the agent to form a capsule preparation. The capsule may be preferably administered by inhalation through an inhalation device generally used. In particular, it is preferable that the active ingredient of the present invention is pulverized into fine particles, adhered to the surfaces of carrier particles of erythritol and trehalose to form a powder product, which is filled into capsules and administered by inhalation through an inhalation device (see, for example, the pamphlet of WO01/026630). Alternatively, it is preferred that an aerosol agent is prepared or a liquid agent of the active ingredient is nebulized by a nebulizer and administered by inhalation.
The dose of the active ingredient of the present invention to humans varies depending on the symptom, age and weight of the patient, therapeutic effect, administration method, and administration period. For example, when the active ingredient is orally administered, the dose suitably falls within the range of 1 mg to 1 g per adult per day. When the active ingredient is administered by inhalation, the dose suitably falls within the range of 0.1 mg to 50 mg per adult per day.

The present invention will be described more specifically below by way of Examples; however, the following will not be construed as limiting the scope of the invention.

### Example 1

Suppressive effect of KP-496 on migration of human neutrophils
1. Method
   a) Preparation of human leukocytes
      Leukocytes were collected from human peripheral blood samples (20 mL of venous blood was taken by a doctor) were obtained from 4 healthy adults (male). They received sufficient information on the purpose of the experiment and gave informed consent in advance. An equivalent amount of 3% dextran (M.W. 208000) saline solution was added to the heparinized blood thus obtained and the mixture was kept to stand for one hour. Thereafter, the supernatant was collected and centrifuged at 150 g for 5 minutes at 4°C. The supernatant was discarded and the pellet was repeatedly twice haemolysed. To explain in detail, 4 mL of an ice-cooled 0.2% NaCl solution was added, quickly suspended and allowed to stand for 20 to 30 seconds. Thereafter, 4 mL of an ice-cooled 1.6% NaCl solution was added to the suspension solution to return the solution to the isotonic condition. After centrifugation was performed at 150 g for 5 minutes at 4°C, the same treatment was repeated. The cell pellet obtained was finally suspended in a buffer (Hank's solution containing 0.1% FBS) and subjected to an experiment.

b) Induction of migration
   This experiment was performed using a 48-well modified Boyden chamber (Neuro Probe). To explain in detail, the wells of the bottom plate of the chamber were filled with 25 µl of vehicle solution containing 10 nmol/L of LTB₄. A polycarbonate filter (PVP-free, pore size: 3 µm) was placed on the bottom plate. The top plate of the chamber was mounted and each well of the upper plate was filled with 45 µL of a leukocyte suspension solution (1.0 × 10⁶ cells /mL) prepared with the buffer. Immediately after that, 5 µL of a test substance (10 fold concentration of a final concentration) was added to the wells. The chamber was incubated in a culture incubator (37°C, 5% CO₂-95% air) for 1.5 hours. After the incubation, the cells (unmigrated cells) present on the top-plate side of the filter were cleaned up. Then the cells that migrated to the bottom-plate side of the filter were fixed in methanol for 5 minutes. The migrated cells were stained with Diff-Quick solution, air-dried on a slide glass and mounted with Entellan new. The cells completely passed through the filter were observed by high-power microscope. The numbers of cells were counted in 4 visual fields (x 400) per well and the average of them was regarded as the number of migrated cells. Each test substance was evaluated using 3 wells. The average value of these wells was calculated and regarded as the value of each substance.

c) Statistical analysis
   The average and standard deviation of each group were calculated. Comparison between the non-treated control group and the LTB₄-treated control group was performed by the Levene's test for equal variance, followed by the t-test. To perform comparison between the LTB₄-treated control group and each of test substance-treated groups, first equal variance was confirmed by the Levene's test and then one-way ANOVA was performed. When the significance level (p value) between groups was less than 0.05, multiple comparisons were performed in accordance with the Dunnett's test. The significance level was set at 0.05%.

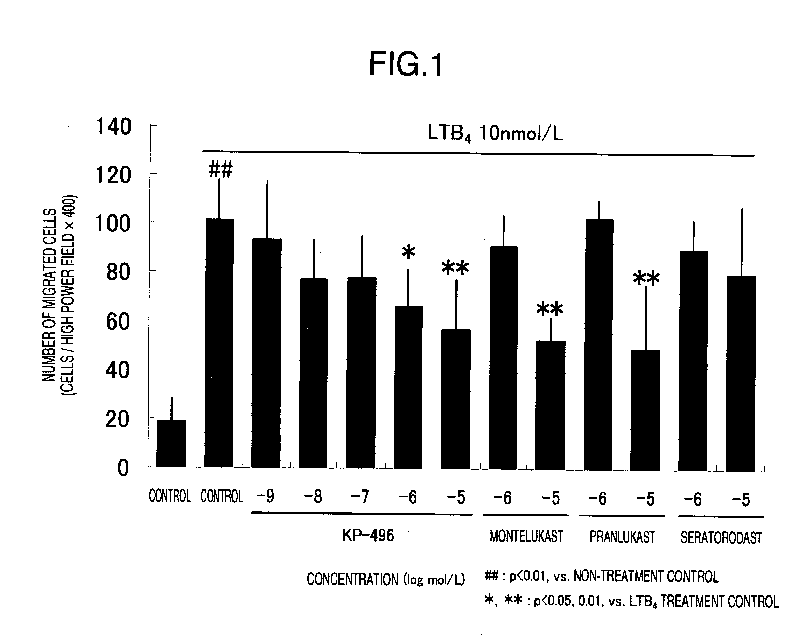

2. Results
   The leukocytes of the human peripheral blood prepared herein contained 78.3 ± 9.7% of neutrophils, 6.5 ± 2.7% of eosinophils, 9.8 ± 6.6% of lymphocytes, and 5.6 ± 2.6% of monocytes, in average. Microscopic observation showed that the cells migrated by LTB₄ treatment were neutrophils alone. KP-496 suppressed the cell migration in a concentration dependent manner and significantly suppressed at a concentration of 1 µmol/L. LTD₄ receptor antagonists, montelukast and pranlukast, also suppressed migration at a concentration of 10 µmol/L; however, did not suppress at all at a concentration of 1 µmol/L. A TXA₂ receptor antagonist, seratorodast, did not suppress the migration at all even at a concentration of 10 µmol/L (Fig. 1).

3. Discussion and conclusion
   KP-496 suppressed LTB₄-induced migration of neutrophils in a concentration dependent manner in a migration test using human leukocytes. The effect of an anti LTD₄ agent was not observed in low concentrations and the effect of an anti TXA₂ agent was not observed even in high concentrations, suggesting that KP-496 suppresses activation and infiltration of inflammatory cells via a mechanism other than those of anti LTD₄ and anti TXA₂.

### Example 2

### Pharmacological effect of KP-496 on interstitial pneumonia and pulmonary fibrosis

1. Method of experimental BLM-induced pulmonary fibrosis model in mice
   a) Induction of animal model of disease and medicament administration
      Bleomycin chloride (BLM) was dissolved in a physiological saline solution (saline). BLM was intravenously administered to 10-week-old ICR-strain male mice once in a dose of 150 mg/10 mL/kg to induce disease. To a non-BLM administration group, saline was administered, instead. KP-496 was administered by inhalation twice a day from the initiation date of BLM administration to the day before autopsy. To describe in brief, a mouse housed in a polyethylene container under unrestrained conditions was allowed to spontaneously inhale a mist state of KP-496 solution (0.5 w/v%) nebulized by a nebulizer for 30 minutes. Furthermore, the vehicle (saline) for KP-496 was administered by inhalation to a control group, in the same manner. A group consisted of 8 animals.

b) Evaluation of bronchoalveolar lavage (hereinafter referred to as "BAL") fluid
   On the 7th day or 21st day after administration of BLM, the mouse was killed by exsanguinations under anaesthesia with pentobarbital. Immediately after that, BAL of the right lung alone was done. BAL fluid (BALF) was centrifuged and the supernatant was removed, and thereafter, precipitated cells were resuspended in saline. The total number of cells was determined by use of a particle counter analyzer. Furthermore, using the remaining cell suspension solution, a smear sample of cells was prepared and stained with Giemsa. The cells were classified according to the cell type. Based on the percentages of cell types, the numbers of classified cells were calculated.

c) Measurement of the content of hydroxyproline in the lung
   After BAL, the right lung was lyophilized. The content of hydroxyproline in the lung tissue was measured in accordance with the KISO method using chloramine T (lnayama et al., Keio J. Med., 27: 43 (1978)).

d) Histopathologic evaluation of the lung
   After BAL, the left lung was fixed in 10 vol% neutral-buffered formalin solution. Thereafter, a paraffin section was prepared in a general way, stained with H. E. and azan and subjected to histopathologic examination of the lung. When an abnormality was observed, the degree of the abnormality was arbitrarily evaluated under blind conditions and scored based on the following 4 stages:
   0: No abnormality is observed
   1: Pathologic lesion is locally observed in a limited area of a sample
   2: Pathologic lesion is observed about a 1/3 region of a sample
   3: Pathologic lesion is observed about a 1/2 region of a sample
   4: Pathologic lesion is observed in substantially the entire region of a sample.

e) Statistic analysis
   The average value and standard deviation of each group were obtained. Comparison between two groups, that is, between BLM administration/vehicle group and BLM administration/KP-496 group or a saline administration/vehicle group, was performed with the Student's t-test. Furthermore, histopathologic lesion scores were evaluated by the Mann-Whitney test. The significance level (P) was set at 0.05%.

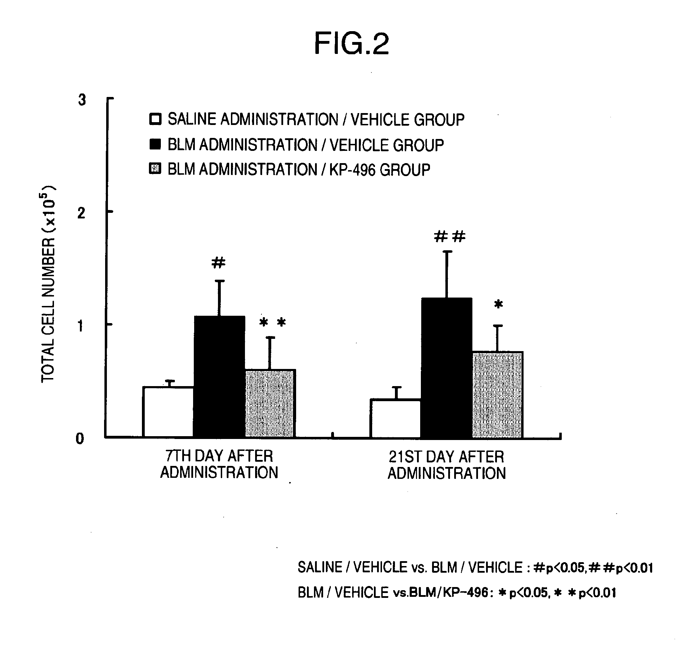

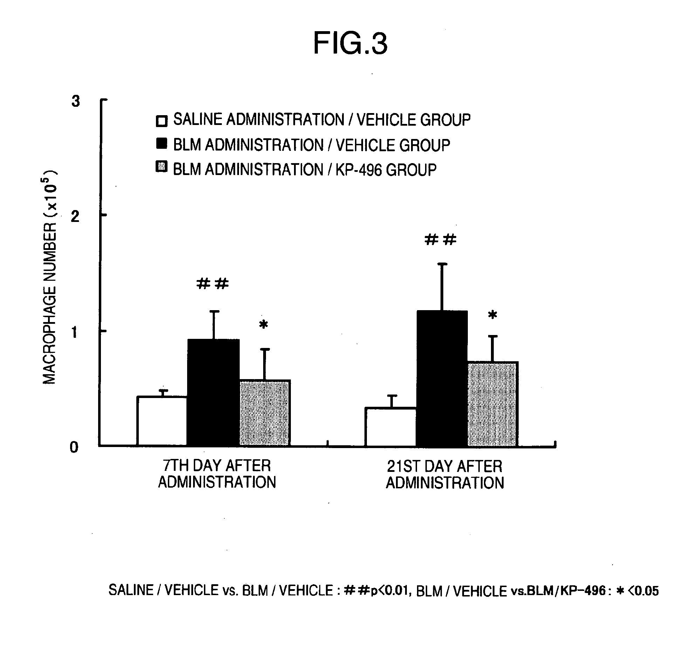

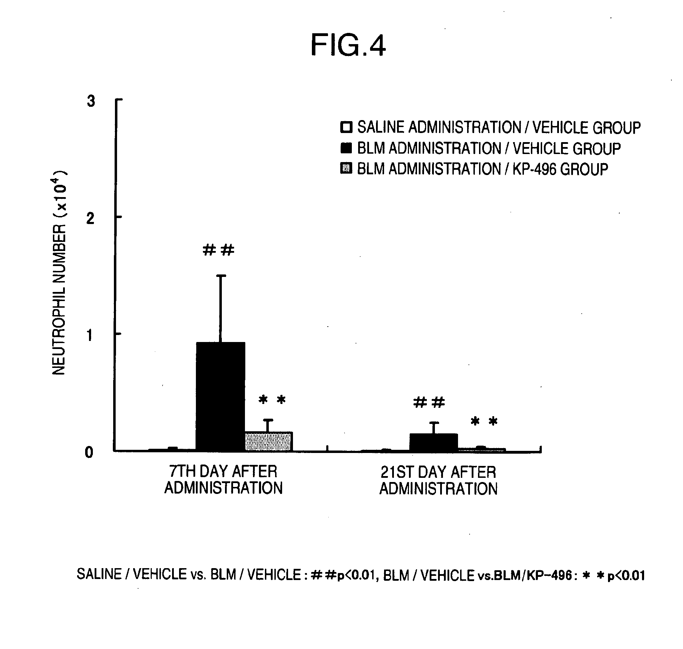

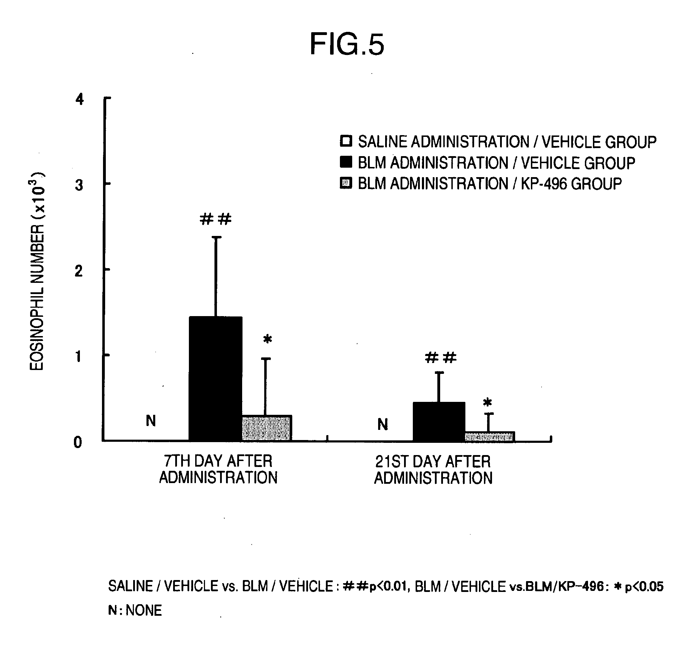

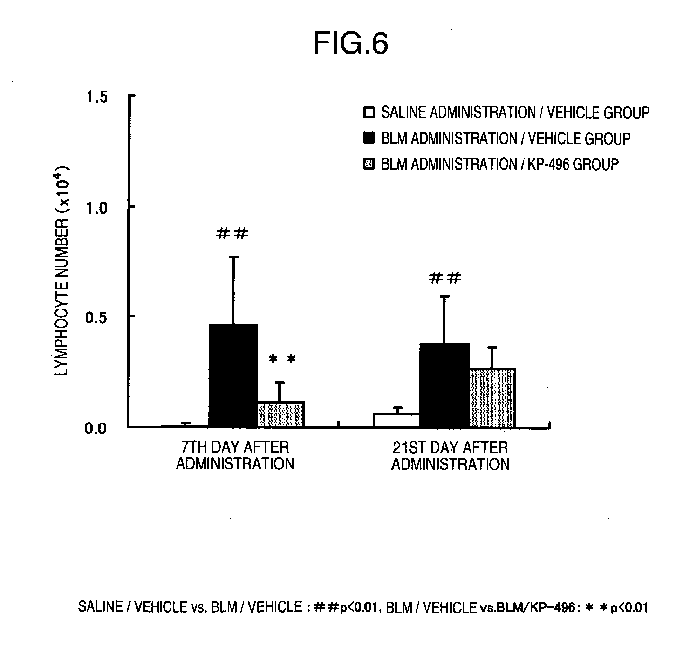

2. Results
   a) Total number of cells in BALF and number of each type cell
      The total numbers of cells in BALF on the 7th day after administration in the BLM administration/vehicle group and saline administration/vehicle group were 1.1 ± 0.31 ×10⁵ cells and 0.4 ± 0.06 × 10⁵ cells, respectively. The BLM administration/vehicle group exhibited a significant increase compared to the saline administration/vehicle group. The BLM administration/KP-496 group, whose cell number was 0.6 ± 0.29 × 10⁵ cells, exhibited a significant decrease compared to the BLM administration/vehicle group (Fig. 2). In view of cell types, alveolar macrophages (0.4 ± 0.05 × 10⁵ cells) were mostly observed in the saline administration/vehicle group. As for other cell types, neutrophils (0.01 ± 0.02 × 10⁴ cells) and lymphocytes (0.01 ± 0.01 × 10⁴ cells) were slightly observed. In the BLM administration/vehicle group, the cell numbers of alveolar macrophages, neutrophils, eosinophils and lymphocytes were 0.9 ± 0.25 × 10⁵ cells, 0.9 ± 0.57 × 10⁴ cells, 1.4 ± 0.93 × 10³ cells and 0.5 ± 0.31 × 10⁴ cells, respectively. All of them exhibited significant increases compared to those of the saline administration/vehicle group. In the BLM administration/KP-496 group, the cell numbers of alveolar macrophages, neutrophils, eosinophils and lymphocytes were 0.6 ± 0.27 × 10⁵ cells, 0.2 ± 0.11 × 10⁴ cells, 0.3 ± 0.68 × 10³ cells and 0.1 ± 0.09 × 10⁴ cells, respectively. All of them exhibited significant decreases compared to those of the BLM administration/vehicle group (Figs. 3, 4, 5 and 6).

The total numbers of cells in BALF on the 21st day in the BLM administration/vehicle group and saline administration/vehicle group were 1.2 ± 0.42 × 10⁵ cells and 0.3 ± 0.12 × 10⁵ cells, respectively. The BLM administration/vehicle group exhibited a significant increase compared to the saline administration/vehicle group. BLM administration/KP-496 group, whose cell number was 0.8 ± 0.24 × 10⁵ cells, exhibited a significant decrease compared to the BLM administration/vehicle group (Fig. 2). In view of cell types, in the saline administration/vehicle group, alveolar macrophages (0.3 ± 0.11 × 10⁵ cells) were mostly observed. As for other cell types, neutrophils (0.01 ± 0.01 × 10⁴ cells) and lymphocytes (0.1 ± 0.02 × 10⁴ cells) were slightly observed. In the BLM administration/vehicle group, the cell numbers of alveolar macrophages, neutrophils, eosinophils and lymphocytes were 1.2 ± 0.40 × 10⁵ cells, 0.2 ± 0.10 × 10⁴ cells, 0.5 ± 0.35 × 10³ cells and 0.4 ± 0.22 × 10⁴ cells, respectively. All of them exhibited significant increases compared to those of the saline administration/vehicle group. In the BLM administration/KP-496 group, the cell numbers of alveolar macrophages, neutrophils, eosinophils and lymphocytes were 0.7 ± 0.23 × 10⁵ cells, 0.02 ± 0.02 × 10⁴ cells, 0.1 ± 0.21 × 10³ cells and 0.3 ± 0.10 × 10⁴ cells, respectively. Alveolar macrophages, neutrophils and eosinophils exhibited significant decreases compared to those of the BLM administration/vehicle group (Figs. 3, 4, 5 and 6).

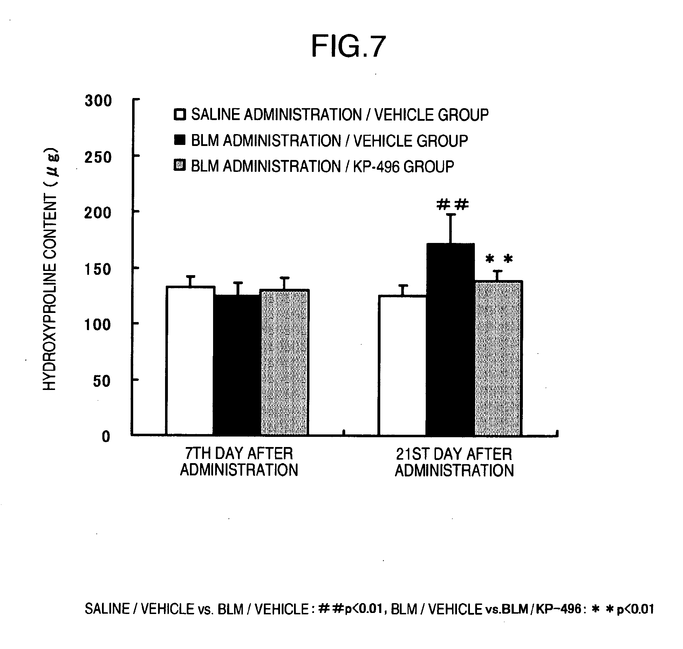

b) The hydroxyproline content of the right lung lobe
   The hydroxyproline contents of the BLM administration/vehicle group and the saline administration/vehicle group on the 7th day after administration were 125.6 ± 10.81 µg and 132.3 ± 9.54 µg, respectively. No difference was observed between the two groups. The BLM administration/KP-496 group exhibited 129.9 ± 11.35 µg. No difference was observed compared to the BLM administration/vehicle group (Fig. 7) .
   The hydroxyproline contents of the BLM administration/vehicle group and the saline administration/vehicle group on the 21st day were 171.6 ± 26.37 µg and 125.5 ± 9.25 µg, respectively. The BLM administration/vehicle group exhibited a significant increase compared to the saline administration/vehicle group. In contrast, the BLM administeration/KP-496 group exhibited 138.0 ± 9.93 µg, exhibiting a significant decrease compared to the BLM administration/vehicle group (Fig. 7).

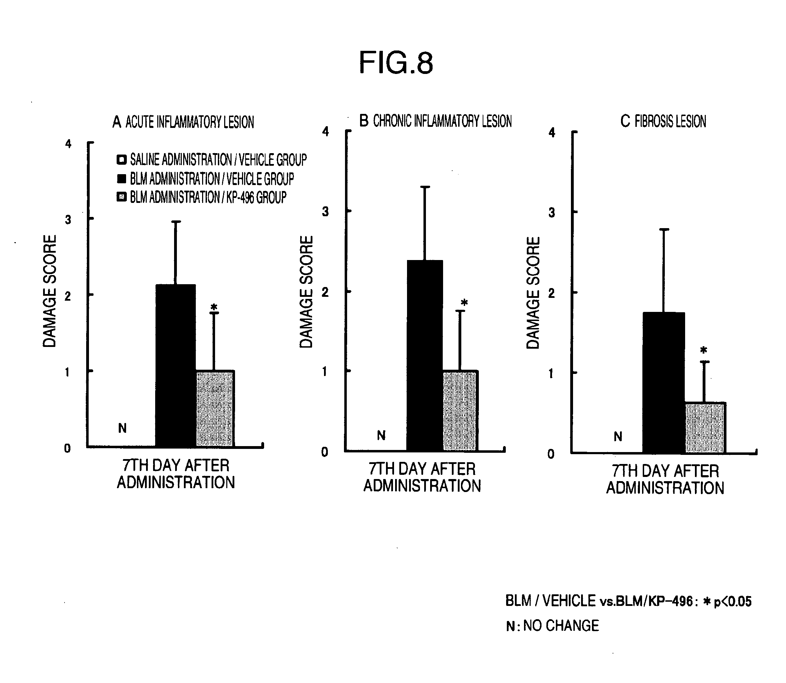

c) Histopathologic evaluation of the left lung lobe
   Histopathologic abnormality of the lung was not observed on the 7th and 21st day in the saline administration/vehicle group. On the other hand, in the BLM administration/vehicle group, infiltration of neutrophils and alveolar macrophages into the alveolar septum and alveolar space, and acute inflammatory changes such as congestion and hemorrhage were observed on the 7th day after administration. The score was 2.1 ± 0.83 (Fig. 8). On the 21st day, the acute inflammation disappeared and changed to chronic inflammations such as enlargement/hyperplasia of alveolar epithelial cells and accumulation of lymphocytes. The score was 2.4 ± 0.92 (Fig. 8). Furthermore, fibrogenesis was observed in the alveolar septum and alveolar space. The score was 1.8 ± 1.04. In contrast, the scores of acute inflammation, chronic inflammation and fibrogenesis in the BLM administration/KP-496 group were 1.0 ± 0.76, 1.0 ± 0.76 and 0.6 ± 0.52, respectively. All of the scores significantly reduced compared to the BLM administration/vehicle group (Fig. 8).

d) Discussion and conclusion
   The present inventors investigated using a mouse model of single-administration BLM-induced pulmonary fibrosis as to whether inhalation administration of KP-496 produced an effect on interstitial pneumonia and pulmonary fibrosis. Inhalation administration was selected as an administration route in order to apply a medicament directly to the lung tissue in which inflammation was to be induced by BLM administration. As a result, KP-496 clearly suppressed a significant increase of the total number of cells in BALF observed both on the 7th and 21st day after BLM administration. In view of cell types in BALF, infiltration of the inflammatory cells such as macrophages, neutrophils, eosinophils and lymphocytes was remarkably observed by BLM administration. KP-496 significantly suppressed the infiltration of the cells. It is noteworthy that KP-496 suppressed infiltration of neutrophils, which is hardly suppressed by steroids.

In the histopathologic findings of the lung, on the 7th day after BLM administration, infiltration of neutrophils and alveolar macrophage, and congesion and hemorrhage, etc., were remarkably observed in the alveolar septum and alveolar space, presenting an acute interstitial pneumonia pathology. Thereafter, on the 21st day, acute interstitial pneumonia disappeared and changed to chronic inflammations such as enlargement/hyperplasia of alveolar epithelial cells and accumulation of lymphocytes; at the same time fibrogenesis occurred in the alveolar septum and space. The lung tissues apparently developed into fibrosis. Furthermore, the content of hydroxyproline, a specific marker of fibrosis significantly increased in the lung tissue. In these histopathologic changes of the lung tissue induced by BLM, KP-496 drastically reduced acute inflammation and chronic inflammation, and inhibited progress to the final pathology of the disease model, i.e., fibrosis. Moreover, KP-496 inhalation clearly decreased the content of hydroxyproline in the lung tissue. From the results, it appeared that KP-496 exhibited an antagonistic action on the activities of lipid mediators such as pLT and TXA₂ produced by inflammatory reactions caused by the pulmonary capillary endothelial cells and alveolar epithelial cells stimulated by BLM administration, thereby reduced early-stage acute inflammation and suppressed the following chronic inflammation and further fibrogenesis.
The results mentioned above demonstrated that inhalation administration of KP-496 to the lung alone is effective as a prevention/therapeutic agent for acute and chronic interstitial pneumonia or pulmonary fibrosis.

### Example 3

### Pharmacological effect of KP-496 on pulmonary emphysema

1. Method of experimental PPE-induced pulmonary emphysema model in mice
   a) Induction of animal model of disease and medicament administration
      Eight-week old C57BL strain mice were anaesthetized with isoflurane. PPE (25 µg) was administered once to the lung through a cannula inserted into the trachea through the pharyngolarynx to induce a pulmonary damage. To a non-PPE administration group (normal group), saline was administered instead. In a preventive administration experiment (hereinafter referred to as a "preventive experiment") of KP-496, drug effect was evaluated on inflammatory cells in BALF 2 days after PPE administration and by histopathological examination of the lung after 10 days. In a therapeutic administration experiment of KP-496 (hereinafter referred to as a "therapeutic experiment"), the drug effect was evaluated by histopathological examination of the lung and weight of the organs 14 days after PPE administration.

The medicament administration period was set from the PPE administration date to the day before autopsy in the preventive experiment and set from a predetermined day (KP-496A group: PPE administration date, KP-496B group: 3 days after PPE administration date, KP-496C group: 7 days after PPE administration date) to the day before autopsy in the therapeutic experiment. Inhalation administration of KP-496 was performed in the same manner as in Example 2. Furthermore, the normal group and the control group were allowed to spontaneously inhale saline, instead. A medicament for use in comparison, prednisolone, was orally administered in a dose of 5 mg/kg twice a day. A group consisted of 10 animals.

b) BAL and drug effect evaluation
   The animals were anaesthetized with pentobarbital and killed by exsanguination.
   Immediately after that, BAL of the right and left lungs was performed with 2 ml of saline. In the same manner as in Example 2, the total number of cells and the number of cells according to the cell type in the BALF were determined.

c) Histopathologic evaluation of the lung
   The lung was excised out from the animal killed by exsanguination in the same manner as in the above, the weight of the lung was measured. The lung was fixed in 10 vol% neutral buffered formalin solution. A pathological tissue sample was prepared in accordance in a general way. The degree of the damage of the lung tissue observed was evaluated by scoring in the same manner as in Example 2. Furthermore, the average of alveolar-space area of the entire lung tissue sample was computationally measured with image-processing software.

d) Statistical analysis
   The average value and standard deviation of each group were calculated. Comparison between the normal group and the control group was performed by the Levene's test for equal variance and then, the t-test was performed depending on the results. To compare the control group and test substance administration groups, equal variance was first confirmed by the Levene's test and then multiple comparisons were performed in accordance with the Dunnett's test. Note that evaluation parameters with unequal variance and the damage score of the lung tissue were analyzed by nonparametric Mann-Whitney test. The significance level employed was 0.05%.

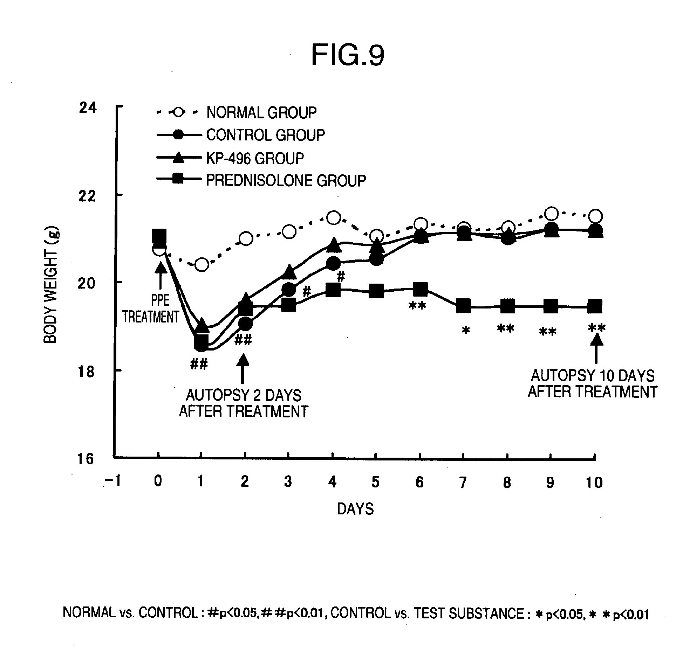

2. Results
   a) Body weight change
      In the preventive experiment, the normal group exhibited a slight reduction of body weight the next day after the saline administration; however, thereafter it showed a mild increasing trend. In contrast, the control group exhibited a significant body weight reduction the next day after PPE administration, and a significant suppression of an increase by 4 days after the administration, compared to the normal group. Thereafter, that is, on and after the 5th day, the control group exhibited virtually the same increase as the control group and thus had the same body weight as the normal group 10 days after the administration. The KP-496 group exhibited substantially the same body weight change as the control group. In the prednisolone administration group, the increase in body weight was strongly suppressed on and after the 3rd day after administration and a significant reduction compared to the control group was observed from the 6th day to 10th day (Fig. 9).

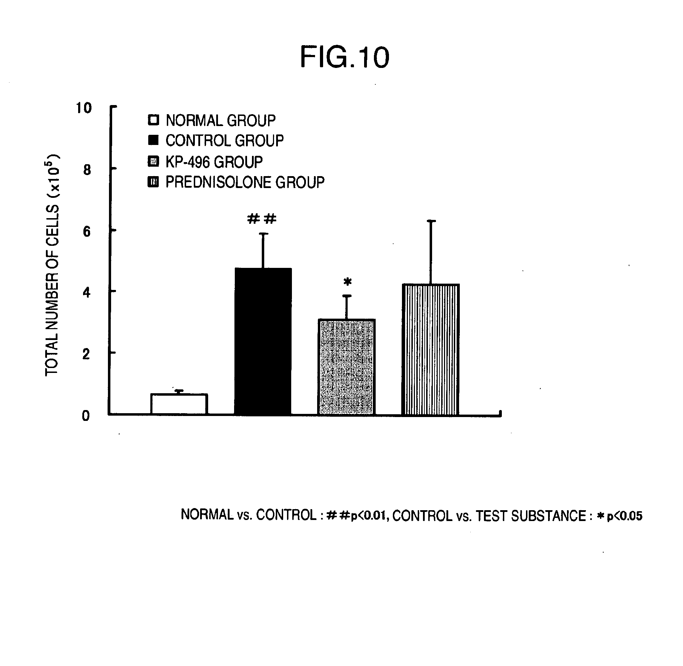

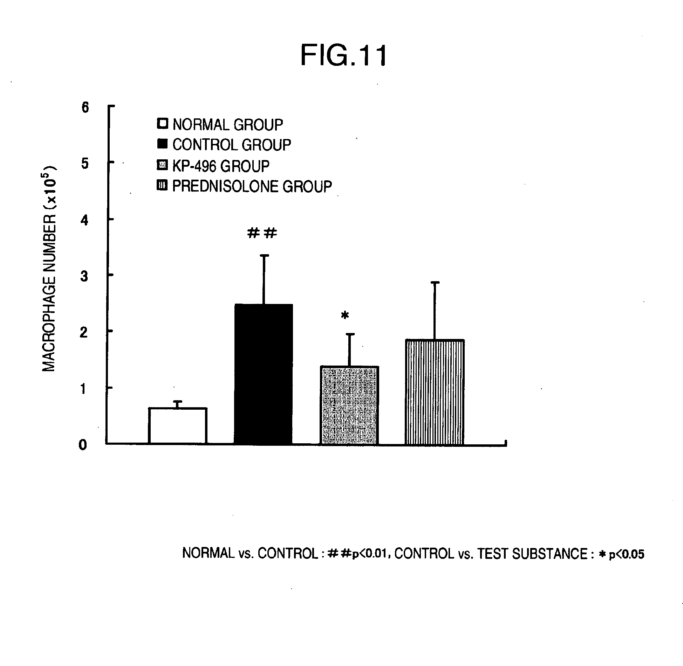

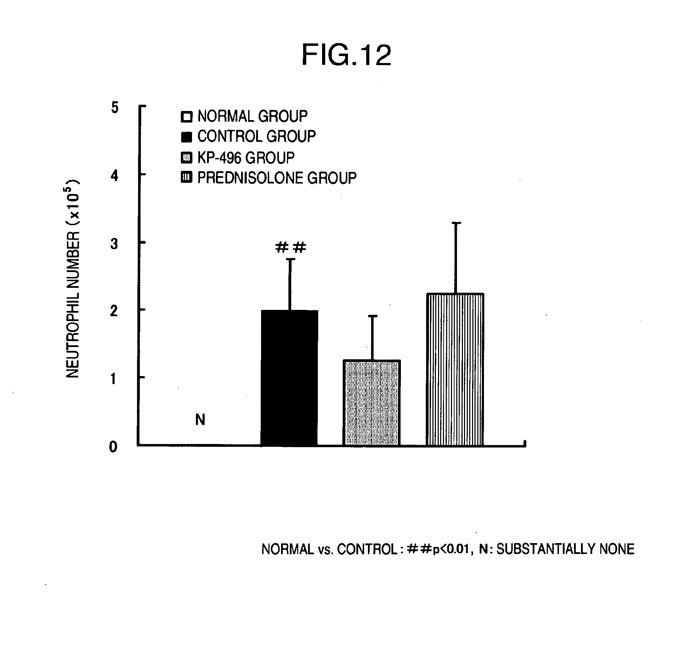

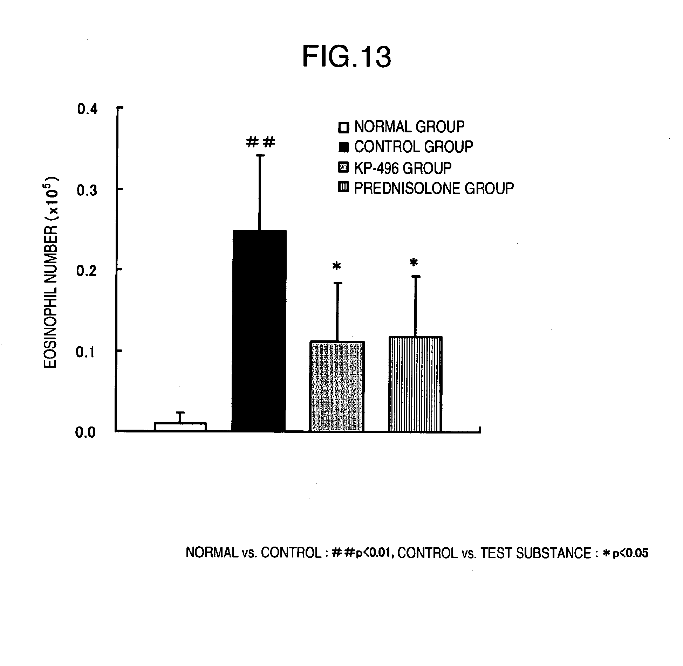

b) Total number of cells and the number of cells according to cell type in BALF
   The total numbers of cells of the normal group and the control group 2 days after administration were 0.7 ± 0.1 × 10⁵ cells and 4.8 ± 1.2 × 10⁵ cells, respectively. The number of cells of the control group significantly increased compared to that of the normal group. KP-496 group had 3.1 ± 0.8 × 10⁵ cells, which were significantly low compared to the control group. On the other hand, the prednisolone group had 4.3 ± 2.1 × 10⁵ cells, which were equivalent to those of the control group (Fig. 10).
   In view of cell type, macrophages occupied the majority of the cells in the normal group. The numbers of the neutrophils and eosinophils were extremely low (Figs. 11, 12 and 13). In the control group, macrophages, neutrophils and eosinophils significantly increased compared to the normal group. In contrast, in the KP-496 group, all of them exhibit lower values than in the control group and macrophages and neutrophils significantly decreased. On the other hand, in the prednisolone group, the number of eosinophils alone was significantly decreased compared to that of the control group.

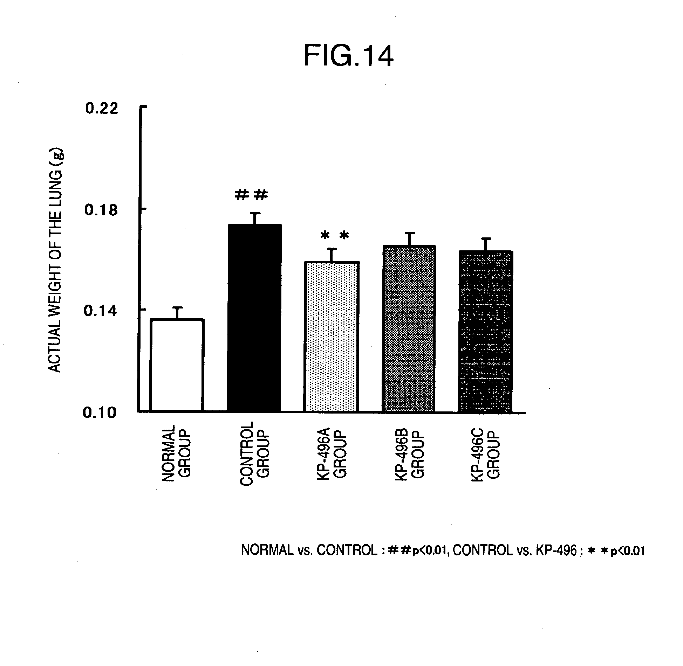

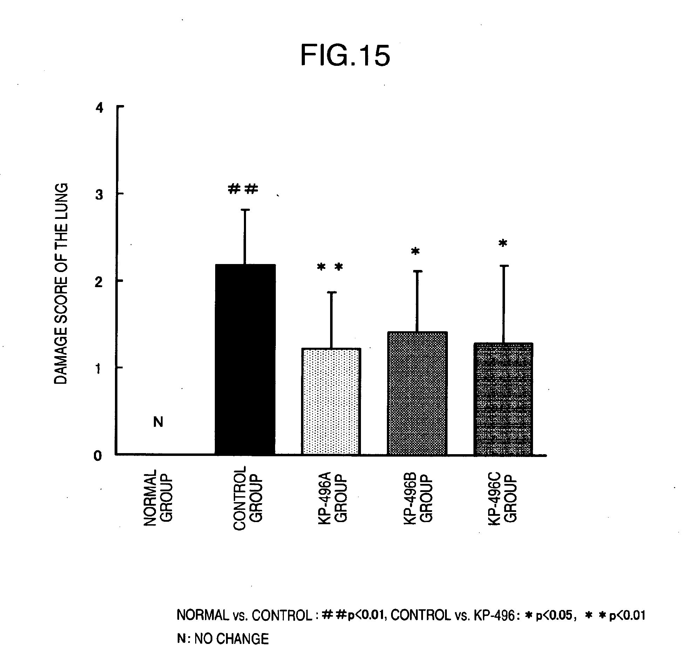

c) Weight of the lung, histopathologic evaluation of the lung and damage score of the lung.
   In the preventive experiment, the lung weight of the control group significantly increased compared to that of the normal group. In contrast, in KP-496 and prednisolone groups, the increase in lung weight was significantly suppressed. In the therapeutic experiment, the KP-496 group exhibited lower values than the control group despite difference of the administration initiation date. In particular, a significant difference was observed between the control group and the group in which administration of KP-496 was started from PPE administration date (Fig. 14).
   In the preventive experiment, any abnormality was not observed at all in the lung of the normal group 10 days after administration. On the other hand, in the control group, the distal airway and the alveolar space significantly enlarged and the lung tissue changed into emphysema-like pathology. In contrast, in the KP-496 group, although the emphysema-like pathology was observed, the pathologic lesion was localized and mild. Furthermore, the control group showed chronic inflammatory pathology such as infiltration of macrophages and small round cells to the alveolar septum and hyperplasia of alveolar epithelial cells. The damage score of the lung significantly increased compared to the normal group. Compared with the control group, KP-496 and prednisolone each significantly decreased the damage score of the lung. Even in the therapeutic experiment, KP-496 significantly decreased the damage score of the lung in each of the groups (Fig. 15).

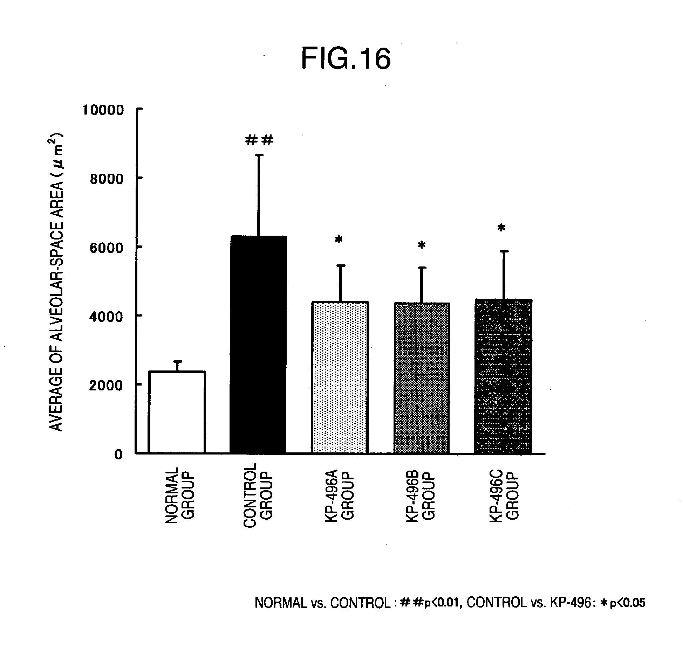

e) Average of the alveolar-space area
   In the preventive experiment, the average of the alveolar-space area in the control group significantly increased compared to that in the normal group. Compared to the control, both of KP-496 and prednisolone significantly suppressed the increase of the average of the alveolar-space area. Even in the therapeutic experiment, KP-496 significantly suppressed the increase of the average of the alveolar-space area in each of the groups (Fig. 16).

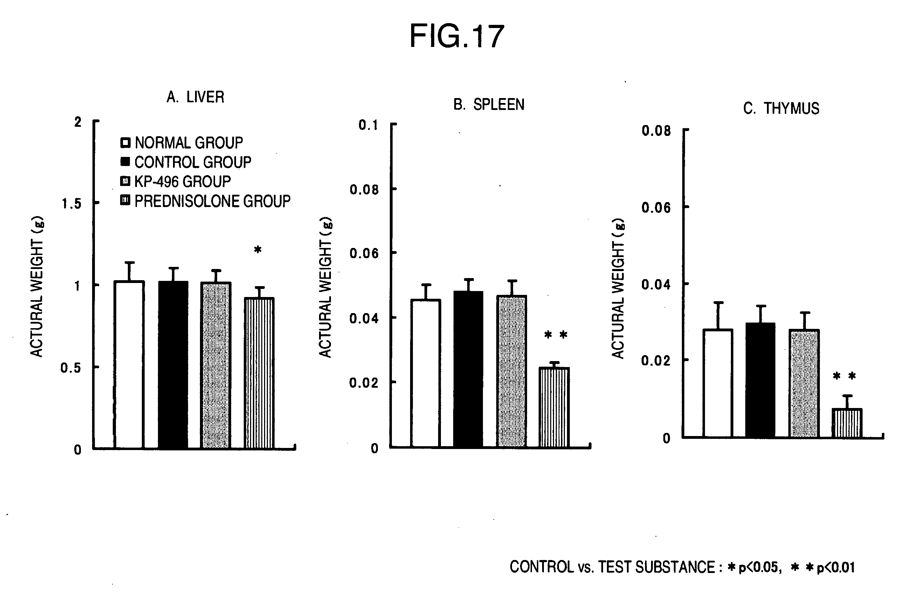

f) Weight of the organs
   No effect was observed on the organ weights in the KP-496 group. In contrast, the weights of the liver, spleen and thymus gland significantly decreased in the prednisolone group compared to those of the control group (Fig. 17).

3. Discussion and conclusion
   KP-496 suppressed acute inflammation caused by PPE and significantly ameliorated progression of emphysema. Since no lesion of emphysema was observed 2 days after PPE administration, such a pulmonary lesion appeared to be not induced directly by PPE through enzymatic action but rather than by the inflammatory cells infiltrated thereto. The ameliorating effect of KP-496 on emphysema was obtained even when it was administered after acute inflammation was over. Therefore, it is suggested that KP-496 inhibits activation of the infiltrated inflammatory cells, resulting in suppression of progression of emphysema. Furthermore, it is inferable that such effects are produced not by acting on the immune organs such as the thymus and spleen, by which prednisolone works, but by suppressing the function of the peripheral inflammatory cells. KP-496 has no effect on the weight of the liver and body weight change and is thus presumably an excellent, safe medicament.

### Example 4

### Pharmacological effect of KP-496 on mucus production

1. Method of experimental model of vanadium pentoxide-induced bronchitis model in mince
   a) Induction of animal model of disease and medicament administration
      Seven-week old C57BL strain male mice were used. The mouse was anaesthetized with isoflurane. Vanadium pentoxide (20 µg) was administered once to the lung through a cannula inserted into the trachea through the pharyngolarynx to induce pathology. To a non-vanadium pentoxide administration group (normal group), saline was administered, instead. KP-496 was administered by inhalation twice a day in the period from the vanadium pentoxide administration date to the day before autopsy, in the same manner as in Example 2. Furthermore, the normal group and the control group were allowed to spontaneously inhale saline in the same manner. A medicament for use in comparison, prednisolone, was orally administered at a dose of 5 mg/kg twice a day in the period from the vanadium pentoxide administration date to the day before autopsy. Note that the normal group consisted of 6 animals and the other group consisted of 10 animals.

b) Measurement of mucus producing region of the intrapulmonary bronchus
   Each of the mice was anaesthetized with pentobarbital 7 days after vanadium pentoxide administration and killed by bloodletting from the abdominal aorta. The lung was excised out and fixed in 10 vol% neutral buffered formalin solution.
   Thereafter, a pathological tissue sample was prepared in a general way and stained with PAS. The area of mucus producing region of the intrapulmonary bronchial epithelial cells was computationally obtained with image analysis software. The obtained value was divided by the circumferential length of the bronchial lumen (obtained in mucosal basal membrane) and the quotient was expressed as a mucus producing region area per millimeter of the bronchus.

c) Statistical analysis
   Statistical analysis was performed in the same manner as in Example 3.

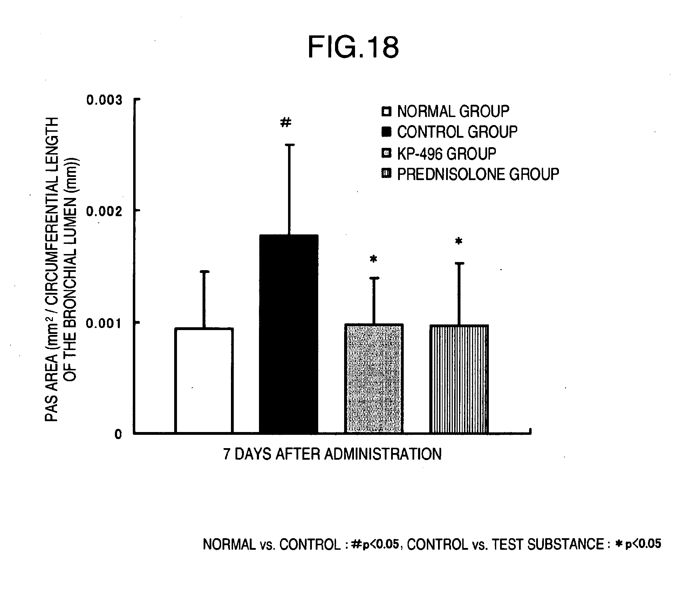

2. Results
   Mucus producing region of intrapulmonary bronchial epithelium
   The PAS positive area of the control group significantly increased compared to the normal group. Compared with the control, both the KP-496 and prednisolone group significantly suppressed the increase of a PAS positive area (Fig. 18).

3. Discussion and conclusion
   KP-496 markedly suppressed excessive secretion of the mucus induced by vanadium pentoxide in the epithelial cells of the intrapulmonary bronchus. Mucus hyperproduction in the bronchial epithelium is one of the characteristics of chronic bronchitis, that is, pathology of COPD. Therefore, it is considered that KP-496 is extremely useful as a therapeutic agent for COPD.

### Example 5

### KP-496 concentration in the mouse lung tissue

1. Method
   Seven-week old C57BL-strain male mice were was used. The mouse was allowed to inhale nebulized KP-496 for 30 min. through a nasal cavity to the lung tissue in the same manner as in Example 2. After completion of the inhalation, the mouse was killed by exsanguination under anaesthesia with pentobarbital. Immediately after that, the lung was excised out, weighed and freezed at -80°C to store. A 4-fold volume of phosphate buffer was added to the lung homogenized and measured with LC-MS. The number of animals was five.
2. Results
   The content of KP-496 in murine lung was 418 ± 114 ng.

3. Discussion and conclusion
   The inhalation dose of KP-496 was about 0.4 µg/mouse. The weight of the mouse was 20 g which means the inhalation dose was 20 µg/kg body weight. Assuming that human weight is 60 kg, the inhalation dose is equivalent to 1 mg/human. It is suggested that chronic inflammatory pulmonary disease can be prevented or treated by the dose.

### INDUSTRIAL APPLICABILITY

The medicament of the present invention containing 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof as an active ingredient is extremely efficient as a preventive or therapeutic agent for acute or chronic interstitial pneumonia or pulmonary fibrosis, and extremely efficient as a preventive or therapeutic agent further for chronic obstructive pulmonary disease such as pulmonary emphysema and chronic bronchitis.

## Claims

1. A medicament for preventing or treating a chronic inflammatory lung disease, containing 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof as an active ingredient.

2. The medicament according to Claim 1, wherein the chronic inflammatory lung disease is interstitial pneumonia or pulmonary fibrosis.

3. The medicament according to Claim 2, wherein the interstitial pneumonia or pulmonary fibrosis is induced as a side effect of chemotherapy.

4. The medicament according to Claim 1, wherein the chronic inflammatory lung disease is chronic obstructive pulmonary disease.

5. The medicament according to any one of Claims 1 to 4, for administering an active ingredient by inhalation into the lung tissue.

6. Use of 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof for producing a medicament for preventing or treating a chronic inflammatory pulmonary disease.

7. A method of preventing or treating a chronic inflammatory pulmonary disease in mammals including human, comprising administering 2-{N-[4-(4-chlorobenzenesulfonylamino)butyl]-N-{3-[(4-isopropyl-2-thiazolyl)methoxy]benzyl}}sulfamoylbenzoic acid or a pharmaceutically acceptable salt thereof to said mammal.
